(19) Europäisches Patentamt
European Patent Office
Office européen des brevets

(11) **EP 2 459 074 B1**

(12) **EUROPEAN PATENT SPECIFICATION**

(45) Date of publication and mention
of the grant of the patent:
**11.07.2018 Bulletin 2018/28**

(21) Application number: **10805129.3**

(22) Date of filing: **30.07.2010**

(51) Int Cl.:
***A61B 8/14*** (2006.01)

(86) International application number:
**PCT/US2010/043971**

(87) International publication number:
**WO 2011/014804 (03.02.2011 Gazette 2011/05)**

(54) **METHODS AND APPARATUS FOR DETECTING AND MAPPING TISSUE INTERFACES**

VERFAHREN UND VORRICHTUNG ZUM ERFASSEN UND ABBILDEN VON
GEWEBESCHNITTSTELLEN

PROCÉDÉS ET APPAREIL DE DÉTECTION ET DE CARTOGRAPHIES D'INTERFACES DE TISSU

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **31.07.2009 US 533307**

(43) Date of publication of application:
**06.06.2012 Bulletin 2012/23**

(73) Proprietor: **St. Jude Medical, Atrial Fibrillation
Division, Inc.**
**St. Paul, Minnesota 55117-9913 (US)**

(72) Inventors:
• **SHAQUER, Cem**
**Los Gatos,**
**California 95032 (US)**
• **SLIWA, John W.**
**Los Altos Hills,**
**California 94024 (US)**
• **MA, Zhenyi**
**San Jose,**
**California 95133 (US)**
• **DANDO, Jeremy D.**
**Plymouth,**
**Minnesota 55447 (US)**

(74) Representative: **Kramer Barske Schmidtchen
Patentanwälte PartG mbB
European Patent Attorneys
Landsberger Strasse 300
80687 München (DE)**

(56) References cited:
| | |
|---|---|
| EP-A1- 1 820 464 | WO-A1-94/13208 |
| WO-A1-95/07657 | US-A- 5 373 849 |
| US-A1- 2002 045 810 | US-A1- 2002 072 675 |
| US-A1- 2003 013 958 | US-A1- 2004 073 110 |
| US-A1- 2006 241 576 | US-A1- 2006 270 934 |
| US-B1- 6 285 898 | US-B1- 6 332 089 |

EP 2 459 074 B1

**Description**

BACKGROUND OF THE INVENTION

a. Field of the Invention

The instant disclosure relates to tissue mapping. In particular, this disclosure relates to apparatus and methods for detecting, locating, and mapping tissue interfaces, for example to generate maps and models of tissue thickness and optionally indices of patient organ status and/or condition.

Background Art

US 6,285,898 B1 relates to diagnosing and treating diseased hearts based on the interaction between cardiac electro-physiological and cardiac bio-mechanical activity without using an acoustic standoff.

US 2003/013958 A1 relates to systems and methods for three-dimensional mapping and reconstruction of an interior of body organs, such as the heart.

EP 1820464 A1 relates to methods and systems for treatment of conduction disturbances of the heart.

US 5,373,849 relates to a device and method for ultrasonic intraluminal imaging by using an intravascular catheter for imaging a portion of a blood vessel in a plane extending axially from the tip of the catheter.

WO 95/07657 A1 relates to an endocardial mapping and ablation system utilizing a separately controlled steerable ablation catheter with ultrasonic imaging capabilities.

US 2004/073110 A1 relates to medical devices for sensing thickness of cardiac tissue by using no side-looking transducer.

It is well known to utilize images and/or geometric models of a patient's heart chamber(s) in conjunction with cardiac diagnostic or therapeutic procedures, such as ablation procedures to treat atrial fibrillation and other cardiac rhythm disorders. It is also known to provide these images and/or geometric models with electrophysiological data, such as maps of electrical potential, overlaid thereon.

Information concerning the thickness of the tissue being mapped and/or treated may also be useful to a practitioner. Unfortunately, such information is presently not readily available to practitioners. While it is known to estimate thickness information from dual-axis fluoroscopic images or based upon the practitioner's histological experience, these methods are inherently subjective and therefore of reduced accuracy and usefulness to practitioners.

BRIEF SUMMARY OF THE INVENTION

The present disclosure teaches, describes, depicts, and provides methods and apparatus for detecting and measuring the location of tissue interfaces and locations where tissue meets body fluids so that relative or actual tissue thickness can be derived or displayed.

In one embodiment, accurate measurement of tissue thickness enables generation of localized tissue thickness maps based upon objective measurements as opposed to subjective estimates using a variety of localization regimes and imaging modalities.

In another embodiment, a family of devices for accurately measuring tissue thicknesses and associating the measured thicknesses with particular locations or coordinates as data points that can be used for tissue mapping of discrete portions and 3D chamber or modeling global portions of the cardiac anatomy of a subject.

Still another object of the present invention is to provide a system that can generate two-, three-, and four-dimensional maps and graphical representations of tissue thickness from data points that associate tissue thickness measurements with particular locations or coordinates at various times and during various parts of the cardiac cycle, thus allowing, for example, periodic review of the cardiac status and/or thickness of certain parts of a heart of a subject (*e.g.*, the left ventricular free, or lateral, wall). In a variation on this aspect of the invention, a cardiac status or heart failure status index can be used alone or in conjunction with other physiologic parameters and characteristics of a subject to optimize therapy or therapies for the subject. Such measurements can be gated or taken during all or a portion of the cardiac cycle of the subject via EGM or surface ECG or via respiratory cycles. Such measurements can also be obtained at various heart rates or times of day (*e.g.*, diurnal cycle).

According to a first aspect, a device for mapping thickness of anatomical structures includes: an elongate catheter body having a distal end portion; at least one localization element carried by the distal end portion of the elongate catheter body to measure a location of the distal end portion of the elongate catheter body via a localization system; at least one pulse-echo transducer carried by the distal end portion of the elongate catheter body, wherein the at least one pulse-echo transducer is adapted to measure a thickness of a tissue proximate the distal end portion of the elongate catheter body; and a controller that associates the measured location of the distal end portion of the elongate catheter body with the measured thickness of the tissue proximate the distal end portion of the elongate catheter body. The at least one localization element may measure a location of the distal end portion of the elongate catheter body within a non-ionizing localization field, such as a magnetic or electrical localization field. In some aspects, at least three localization elements may be used, for example in order to provide six-dimensional localization (*e.g.*, position and orientation) of the distal end portion of the elongate catheter body.

The at least one pulse-echo transducer may be oriented such that it emits and receives energy in a direction along a longitudinal axis of the elongate catheter body, or, alternatively, such that it emits and receives energy in a direction angled with respect to a longitudinal axis of the elongate catheter body. Of course, multiple pulse-echo transducers may be used, some of which are oriented along the longitudinal axis of the catheter body and some of which are angled relative to the longitudinal axis of the catheter body. It is also contemplated that one or more pulse-echo transducers may be movable (e.g., configured to slide along the elongate catheter body or to rotate within or about the elongate catheter body). According to the invention, a standoff is positioned relative to the at least one pulse-echo transducer such that energy emitted and received by the at least one pulse-echo transducer travels through the at least one standoff. Furthermore, the distal end portion of the elongate catheter body is rigid where the at least one pulse-echo transducer is carried.

In another aspect, a system for mapping cardiac tissue thickness includes: a localization system; an elongate catheter body having a distal end portion; at least one localization element carried by the distal end portion of the elongate catheter body to measure a location of the distal end portion of the elongate catheter body within a portion of a heart using the localization system; and at least one ultrasonic transducer carried by the distal end portion of the elongate catheter body, the at least one ultrasonic transducer operable to measure a thickness of a cardiac tissue proximate the distal end portion of the elongate catheter body. The localization system includes a controller that associates the measured location of the distal end portion of the elongate catheter body with the measured thickness of the cardiac tissue proximate the distal end portion of the elongate catheter body as a data point. A memory device may be provided to store a plurality of data points.

In some embodiments, the localization system generates an electric field and the at least one localization element measures a characteristic of the electric field to determine the location of the distal end portion of the elongate catheter body. In other embodiments, the localization system generates a magnetic field and the at least one localization element measures a characteristic of the magnetic field to determine the location of the distal end portion of the elongate catheter body. In still other embodiments, the localization system is an acoustic localization system. In further embodiments, the localization system outputs or provides data for a three-dimensional image of the portion of the heart on a display, and the at least one localization element makes the distal end portion of the elongate catheter body visible within the three-dimensional image. For example, the at least one localization element may be radiopaque. The three-dimensional image may be real-time or pre-recorded. It is also contemplated to generate the three-dimensional model of the portion of the heart from the plurality of data points.

Optionally, the system includes a mapping processor that generates a map of cardiac tissue thickness from a plurality of data points and a display that outputs a graphical representation of the map of cardiac tissue thickness overlaid upon a three-dimensional model of the portion of the heart. The display may also output a graphical representation of electrophysiological data for the portion of the heart overlaid upon the three-dimensional model of the portion of the heart. The thickness map may, for example, be a colorized map. Alternatively or additionally, the map may include alphanumeric and/or synthesized voice reporting of a local thickness on request of the practitioner, for example based on the practitioner identifying a point of interest on the graphical model using a mouse or other suitable input device.

Also disclosed herein is a method of mapping cardiac tissue thickness that includes the steps of: providing a catheter having a distal end portion including at least one localization element and at least one acoustic transducer; introducing the catheter into a portion of a heart (e.g., intravascular introduction); measuring a location of the distal end portion of the catheter utilizing the at least one localization element; measuring a thickness of a cardiac tissue proximate the distal end portion of the catheter utilizing the at least one acoustic transducer; and associating the measured location of the distal end portion of the catheter with the measured thickness of the cardiac tissue as a data point. In some examples the step of measuring a thickness of a cardiac tissue proximate the distal end portion of the catheter includes the following steps: emitting a pulse of acoustic energy from the at least one acoustic transducer towards the cardiac tissue; receiving a first acoustic echo from a first surface of the cardiac tissue at the at least one acoustic transducer; receiving a second acoustic echo from a second surface of the cardiac tissue at the at least one acoustic transducer; and interpreting the first and second acoustic echoes as information about the thickness of the cardiac tissue.

The steps of measuring a location of the distal end portion of the catheter utilizing the at least one localization element; measuring a thickness of a cardiac tissue proximate the distal end portion of the catheter utilizing the at least one acoustic transducer; and associating the measured location of the distal end portion of the catheter with the measured thickness of the cardiac tissue as a data point may be repeated for a plurality of locations within the portion of the heart, thereby generating a plurality of geometry data points. A graphical representation of cardiac tissue thickness may then be overlaid upon a three-dimensional model of the portion of the heart using the plurality of geometry data points.

Alternatively or additionally, the steps of measuring a thickness of a cardiac tissue proximate the distal end portion of the catheter utilizing the at least one acoustic transducer; and associating the measured location of the distal end portion of the catheter with the measured thickness of the cardiac tissue as a data point may be repeated at a single location within the heart chamber a plurality of times to generate a plurality of cardiac cycle data points. A graphical representation of changes in cardiac tissue thickness over time at the measured location of the distal end portion of the catheter may be displayed using the plurality of cardiac cycle data points. It is contemplated that this representation may be overlaid upon a three-dimensional model of the portion of the heart.

Further, in some examples, an average (*e.g.*, mean), minimum, or maximum thickness of the cardiac tissue is derived from the plurality of cardiac cycle data points, allowing the average, minimum, or maximum thickness to be associated with the measured location of the at least one localization element as a geometry data point. A plurality of such geometry data points may be generated, and a graphical representation of average, minimum, or maximum cardiac tissue thickness may be overlaid upon a three-dimensional model of the portion of the heart from the plurality of geometry data points.

In still another aspect, a device for measuring a spatial location of a tissue interface includes: an elongate catheter body having a distal end portion; a plurality of localization elements carried by the distal end portion of the elongate catheter body to localize the distal end portion of the elongate catheter body; at least one pulse-echo acoustic element carried by the distal end portion of the elongate catheter body operable to detect a distance to a tissue interface proximate the distal end portion of the catheter body; and a controller that determines a location of the tissue interface from the localization of the distal end portion of the catheter body and the detected distance to the tissue interface.

In a further aspect, the present disclosure describes a method of mapping a tissue surface including the following steps: providing a catheter having a distal end portion including at least one localization element and at least one acoustic transducer; introducing the catheter into a portion of a heart; localizing the distal end portion of the catheter utilizing the at least one localization element; detecting a distance to a tissue interface using the at least one acoustic transducer; and determining a location of the tissue interface from the localization of the distal end portion of the catheter and the detected distance to the tissue interface. By repeating these steps for a plurality of positions and/or orientations of the distal end portion of the catheter (*e.g.*, by moving the distal end portion of the catheter through the portion of the heart or by allowing the distal end portion of the catheter to "float" within the blood pool), a tissue surface map may be generated for the portion of the heart.

An advantage of the present invention is that it facilitates measuring tissue thicknesses with increased accuracy.

Another advantage of the present invention is that it associates measured tissue thicknesses with particular spatial coordinates. The resultant plurality of data points may be used to generate maps and/or graphical representations of tissue thickness.

Still another advantage of the present invention is that acoustic echo measurements are often more accurate than measurements automatically or manually derived from presurgical images (*e.g.*, CATSCAN or MRI images).

The foregoing and other aspects, features, details, utilities, and advantages of the present invention will be apparent from reading the following description and claims, and from reviewing the accompanying drawings.

BRIEF DESCRIPTION OF THE DRAWINGS

Fig. 1 is a schematic diagram of a localization system representative of the type that may be utilized in connection with the present invention.
Fig. 2 depicts an exemplary catheter that may be used in conjunction with the localization system of Fig. 1.
Fig. 3 is a close up, partially cut-away view of the distal end portion of the catheter depicted in Fig. 2.
Fig. 4 illustrates, in partial cut-away view, the distal end portion of an exemplary circular mapping catheter that may be utilized in some embodiments of the present invention.
Fig. 5 schematically illustrates a catheter according to the present invention in use to measure cardiac tissue thickness.
Fig. 6 is a graph relating amplitudes of received acoustic echoes along a sensing direction to time.

DETAILED DESCRIPTION OF THE INVENTION

The present invention provides a system for locating tissue interfaces and locations where diverse tissue meets other tissue (*e.g.*, tissue having different density or the like) or where body fluids are the dominant adjoining anatomical "structure," for example in order to map tissue or fluid volumes and/or to measure tissue thicknesses. For purposes of illustration, the invention will be described in detail in the context of measuring and mapping cardiac tissue thickness. It is contemplated, however, that the present invention may be practiced to good advantage in other contexts, such as the detection of potential aneurysms via the detection of thinned cardiac tissue or the detection of relatively thick chamber tissue indicating a potentially deleterious progression of heart failure or other circulatory disease. The present invention may also be practiced to good advantage to gather data relating to variations in tissue thickness over time (*e.g.*, thickness vs. heartbeat phase) that relates to the health of the heart.

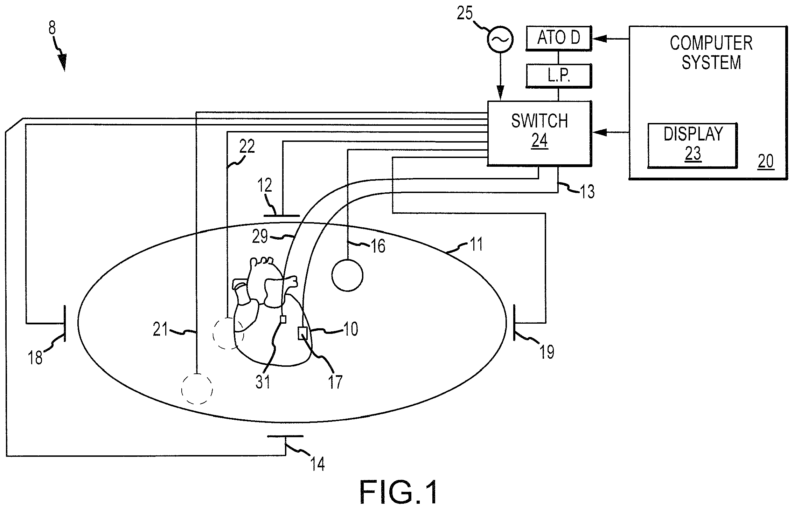

Fig. 1 shows a schematic diagram of a localization system 8 representative of the type of localization system that may be utilized in connection with the present invention. As one of ordinary skill in the art will recognize, and as will be further described below, localization system 8 (which may also sometimes be referred to as a "navigation system" or a "spatial navigation system") determines the position, and optionally the orientation, of objects, typically within a three-dimensional space, and expresses those positions and orientations relative to at least one reference frame. For example, localization systems often express position or location in terms of (x, y, z) coordinates and orientation in terms of angles of rotation about the x-, y-, and z-axis (*e.g.*, $\Theta_1$, $\Theta_2$, $\Theta_3$).

It should be understood that (x, y, z) coordinates locate a point in space within a given reference frame, such as with respect to a solid model of cardiac chambers drawn in that XYZ reference frame. Similarly, $\Theta_1$, $\Theta_2$, $\Theta_3$ describe the orientation of an object located at that (x, y, z) point with respect to that XYZ reference frame. Of course, other coordinate systems (*e.g.*, polar, cylindrical, and spherical coordinate systems) may be used as well.

It should also be understood that localization system 8 may directly measure the orientation of an object within the reference frame. Alternatively, as discussed in detail herein, the orientation of the object within the reference frame may be derived from the measured position of a plurality of localization elements. The term "localize" will be used herein to refer to determining both position and orientation of an object within a localization field, regardless of whether orientation is directly measured or derived from position information.

Localization system 8 may be utilized to conduct a cardiac electrophysiology study, for example by navigating a cardiac catheter and measuring electrical activity occurring in a heart 10 of a patient 11 and three-dimensionally mapping the electrical activity and/or information related to or representative of the electrical activity so measured. Localization system 8 can be used, for example, to create an anatomical model of the patient's heart 10 using one or more locating electrodes. Localization system 8 can also be used to measure electrophysiology data at a plurality of points along a cardiac surface, and store the measured data in association with location information for each measurement point at which the electrophysiology data was measured, for example to create a diagnostic data map of the patient's heart 10. Of course, localization system 8 may also be employed in other diagnostic and/or therapeutic procedures, such as ablation treatments for atrial fibrillation and other rhythm disorders. It should also be understood that a locating electrode and an electrophysiology sensing electrode may, in some embodiments of the invention, be a shared electrode.

For simplicity of illustration, the patient 11 is depicted schematically as an oval. In the embodiment shown in Figure 1, three sets of surface electrodes (*e.g.*, patch electrodes) are shown applied to a surface of the patient 11, defining three generally orthogonal axes, referred to herein as an x-axis, a y-axis, and a z-axis. In other embodiments the electrodes could be positioned in other arrangements, for example multiple electrodes on a particular body surface. Likewise, the electrodes do not need to be on the body surface, but could be fixed on an external apparatus, or electrodes positioned internally to the body could be used.

In Figure 1, the x-axis surface electrodes 12, 14 are applied to the patient along a first axis, such as on the lateral sides of the thorax region of the patient (*e.g.*, applied to the patient's skin underneath each arm) and may be referred to as the Left and Right electrodes. The y-axis electrodes 18, 19 are applied to the patient along a second axis generally orthogonal to the x-axis, such as along the inner thigh and neck regions of the patient, and may be referred to as the Left Leg and Neck electrodes. The z-axis electrodes 16, 22 are applied along a third axis generally orthogonal to both the x-axis and the y-axis, such as along the sternum and spine of the patient in the thorax region, and may be referred to as the Chest and Back electrodes. The heart 10 lies between these pairs of surface electrodes 12/14, 18/19, and 16/22.

An additional surface reference electrode (*e.g.*, a so-called "belly patch") 21 provides a reference and/or ground electrode for the localization system 8. The belly patch electrode 21 may be an alternative to a fixed intracardiac electrode 31, described in further detail below. It should also be appreciated that, in addition, the patient 11 may have most or all of the conventional 12 electrodes for taking a surface electrocardiogram ("ECG") in place. This ECG information is available to the localization system 8, although not illustrated in Fig. 1.

A representative catheter 13 having an elongate body (*e.g.*, a body of length sufficient to reach the patient's heart when introduced into the patient's vasculature via the femoral artery or vein) and at least one electrode 17 (*e.g.*, a distal electrode) is also shown. An exemplary catheter might have an outer diameter of less than about 12 French, such as between about 7 French and about 8 French, though one of ordinary skill in the art will appreciate that the catheter can be made larger or smaller without departing from the present teachings. This representative catheter electrode 17 is referred to as the "roving electrode," "moving electrode," or "measurement electrode" throughout the specification. Typically, multiple electrodes on catheter 13, or on multiple such catheters, will be used. In one embodiment, for example, localization system 8 may comprise sixty-four electrodes on twelve catheters disposed within the heart and/or vasculature of the patient. Of course, this embodiment is merely exemplary, and any number of electrodes and catheters may be used within the scope of the present invention.

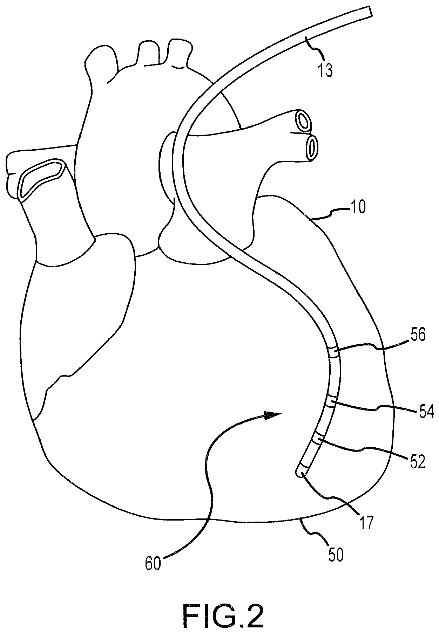

For purposes of this disclosure, an exemplary catheter 13 is shown in Fig. 2. In Fig. 2, catheter 13 extends into the left ventricle 50 of the patient's heart 10. Catheter 13 includes electrode 17 on its distal tip, as well as a plurality of additional measurement electrodes 52, 54, 56 spaced along its length. Typically, the spacing between adjacent electrodes will be known, though it should be understood that the electrodes may not be evenly spaced along catheter 13 or of equal size to each other. Since each of these electrodes 17, 52, 54, 56 lies within the patient, location data may be collected simultaneously for each of the electrodes by localization system 8.

It is contemplated that a "string" of electrodes may be used to measure the curved shape of catheter 13 depicted in Fig. 2. It is also contemplated that two or more closely-spaced electrodes placed near the tip of catheter 13 can report the "pointing direction" (*e.g.*, the orientation) of the tip itself. That is, for electrodes that are sufficiently closely spaced, the measured positions of the individual electrodes (*e.g.*, their (x, y, z) coordinates) can also be used to determine the orientation of that portion of the catheter (*e.g.*, to compute $\Theta_1$, $\Theta_2$, and $\Theta_3$). As described in detail herein, the orientation of catheter 13 is of interest in certain applications, such as where catheter 13 axially emits acoustic energy, such as a distance-sensing axial ultrasonic pinging beam. Typically, at least the blood-contacting portion of catheter 13 is a single-use, disposable component.

Returning now to Fig. 1, an optional fixed reference electrode 31 (*e.g.*, attached to a wall of the heart 10) is shown on a second catheter 29. For calibration purposes, this electrode 31 may be stationary (*e.g.*, attached to or near the wall of the heart) or disposed in a fixed spatial relationship with the roving electrodes (*e.g.*, electrodes 17, 52, 54, 56), and thus may be referred to as a "navigational reference" or "local reference." The fixed reference electrode 31 may be used in addition or alternatively to the surface reference electrode 21 described above. In many instances, a coronary sinus electrode or other fixed electrode in the heart 10 can be used as a reference for measuring voltages and displacements; that is, as described below, fixed reference electrode 31 may define the origin of a coordinate system.

Each surface electrode is coupled to the multiplex switch 24, and the pairs of surface electrodes are selected by software running on a computer 20, which couples the surface electrodes to a signal generator 25. The computer 20, for example, may comprise a conventional general-purpose computer, a special-purpose computer, a distributed computer, or any other type of computer. The computer 20 may comprise one or more processors, such as a single central processing unit, or a plurality of processing units, commonly referred to as a parallel processing environment, which may execute instructions to practice the various aspects of the present invention described herein.

Generally, three nominally orthogonal electric fields are generated by a series of driven and sensed electric dipoles (*e.g.*, surface electrode pairs 12/14, 18/19, and 16/22) in order to realize catheter navigation in a biological conductor. Alternatively, these orthogonal fields can be decomposed and any pairs of surface electrodes can be driven as dipoles to provide effective electrode triangulation. Likewise, the electrodes 12, 14, 18, 19, 16, and 22 (or any number of electrodes) could be positioned in any other effective arrangement for driving a current to or sensing a current from an electrode in the heart. For example, multiple electrodes could be placed on the back, sides, and/or belly of patient 11. Additionally, such non-orthogonal methodologies add to the flexibility of the system. For any desired axis, the potentials measured across the roving electrodes resulting from a predetermined set of drive (source-sink) configurations may be combined algebraically to yield the same effective potential as would be obtained by simply driving a uniform current along the orthogonal axes.

Thus, any two of the surface electrodes 12, 14, 16, 18, 19, 22 may be selected as a dipole source and sink with respect to a ground reference, such as belly patch 21, while the unexcited electrodes measure voltage with respect to the ground reference. The roving electrodes 17, 52, 54, 56 placed in the heart 10 are exposed to the field from a current pulse and are measured with respect to ground, such as belly patch 21. In practice the catheters within the heart may contain more or fewer electrodes than the four shown, and each electrode potential may be measured. As previously noted, at least one electrode may be fixed to the interior surface of the heart to form a fixed reference electrode 31, which is also measured with respect to ground, such as belly patch 21, and which may be defined as the origin of the coordinate system relative to which localization system 8 measures positions. Data sets from each of the surface electrodes, the internal electrodes, and the virtual electrodes may all be used to determine the location of the roving electrodes 17, 52, 54, 56 within heart 10.

The measured voltages may be used to determine the location in three-dimensional space of the electrodes inside the heart, such as roving electrodes 17, 52, 54, 56, relative to a reference location, such as reference electrode 31. That is, the voltages measured at reference electrode 31 may be used to define the origin of a coordinate system, while the voltages measured at roving electrodes 17, 52, 54, 56 may be used to express the location of roving electrodes 17, 52, 54, 56 relative to the origin. Preferably, the coordinate system is a three-dimensional (x, y, z) Cartesian coordinate system, though the use of other coordinate systems, such as polar, spherical, and cylindrical coordinate systems, is within the scope of the invention.

As should be clear from the foregoing discussion, the data used to determine the location of the electrode(s) within the heart is measured while the surface electrode pairs impress an electric field on the heart. The electrode data may also be used to create a respiration compensation value used to improve the "raw location data" for the electrode locations as described in United States patent application publication no. US 2004/0254437 A1. The electrode data may also be used to compensate for changes in the impedance of the body of the patient as described in co-pending United States application no. 11/227,580.

In summary, the localization system 8 first selects a set of surface electrodes and then drives them with current pulses. While the current pulses are being delivered, electrical activity, such as the voltages measured at least at one of the remaining non-selected surface electrodes and *in vivo* electrodes, is measured and stored. Compensation for artifacts, such as respiration and/or impedance shifting, may be performed as indicated above.

In a preferred embodiment, the localization/mapping system is the EnSite NavX™ navigation and visualization system of St. Jude Medical, Atrial Fibrillation Division, Inc., which generates the electrical fields described above. Other localization systems, however, may be used in connection with the present invention, including for example, the MediGuide System owned by St. Jude Medical, Atrial Fibrillation Division, Inc., the CARTO navigation and location system of Biosense Webster, Inc., the AURORA system of Northern Digital Inc., or Sterotaxis' NIOBE Magnetic Navigation System, all of which utilize magnetic fields rather than electrical fields. The localization and mapping systems described in the following patents can also be used with the present invention: United States patent nos. 6,990,370; 6,978,168; 6,947,785; 6,939,309; 6,728,562; 6,640,119; 5,983,126; and 5,697,377.

The fields generated by localization system 8, whether electrical fields (*e.g.*, EnSite NavX™), magnetic fields (*e.g.*, MEDIGUIDE, CARTO, AURORA, NIOBE), or another suitable field, may be referred to generically as "localization fields," while the elements generating the fields, such as surface electrodes 12, 14, 16, 18, 19, and 22 may be generically referred to as "localization field generators." As described above, surface electrodes 12, 14, 16, 18, 19, and 22 may also function as detectors to measure the characteristics of the localization field (*e.g.*, the voltages measured at roving electrodes 17, 52, 54, 56, or a current from roving electrodes 17, 52, 54, 56), and thus may also be referred to as "localization elements." Though the present invention is described primarily in the context of a localization system that generates an electrical field, one of ordinary skill in the art will understand how to apply the principles disclosed herein in other types of localization fields, and in particular other types of non-ionizing localization fields (*e.g.*, by replacing electrodes 17, 52, 54, 56 with coils to detect or transmit differently-oriented components of a magnetic field).

Moreover, the principles disclosed herein can also be applied in connection with other types of localization systems. For example, according to some aspects of the invention, localization system 8 may be an acoustic localization system. One example of an acoustic localization system is the BIRTHTRACK Continuous Labor Monitoring System of Barnev, Inc. Although an acoustic localization system does not generate a "field" in the same sense as the electrical and magnetic localization systems described above, the terms "localization field" and "localization element" will nonetheless be used herein to refer to the analogous aspects of an acoustic localization system. In particular, the term "localization element" includes acoustic emitters and/or receptors that may be carried by catheter 13 in connection with an acoustic localization system.

In still other embodiments of the invention, localization system 8 may employ real-time and/or pre-recorded three-dimensional imaging or imagery. Suitable three-dimensional images include, without limitation, CT images, magnetic resonance images, ultrasound images, x-ray images, fluoroscopic images, and three-dimensional images generated by other suitable imaging modalities or techniques. The three-dimensional images may also be presented as volumetric and/or surface models generated from position data collected using a localization system. For example, United States application no. 11/967,214, provides one suitable method and system for modeling a surface, typically in three dimensions, from an unstructured cloud of geometry points such as may be collected using a localization system. As with acoustic localization systems, the terms "localization field" and "localization element" will be used to refer to the analogous aspects of such systems. For example, the term "localization element" will be used to refer to features, such as radiopaque markers, that make the location of catheter 13 visible in a three-dimensional image.

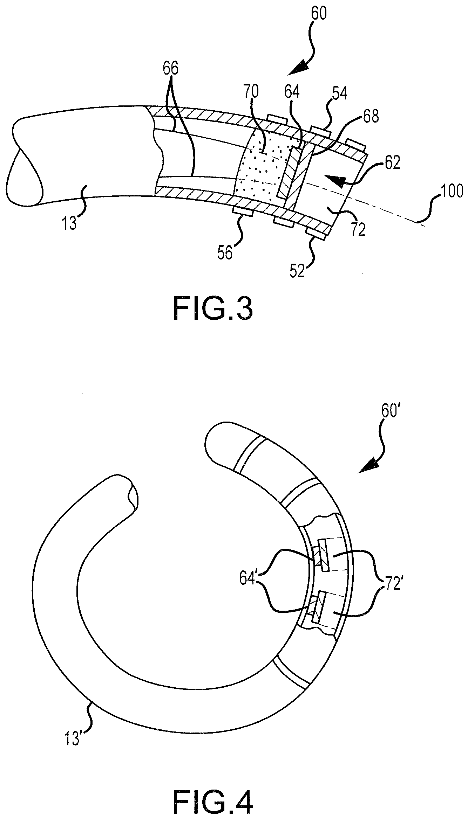

Fig. 3 is a close up and partial cut-away view of the distal end portion 60 of catheter 13 depicted in Fig. 2. As shown in Fig. 3, electrodes 52, 54, and 56 are carried on an exterior surface of distal end portion 60 to enable the localization of distal end portion 60 within a localization field generated by localization system 8. Of course, electrodes 52, 54, and 56, or other suitable localization elements, could be carried internal to distal end portion 60 of catheter 13 as well depending on the particular localization system utilized. For example, when the localization system is the EnSite NavX™ navigation and visualization system of St. Jude Medical, Atrial Fibrillation Division, Inc., electrodes 52, 54, and 56 will be on the exterior of the catheter (*e.g.*, they will be blood-contacting).

Distal end portion 60 further carries at least one tissue thickness measurement element 62 that allows catheter 13 to be used to detect tissue interfaces and map tissue thickness according to the present teachings. As discussed in detail below, tissue thickness measurement element 62 is able to measure the thickness of tissue proximate distal end portion 60 along at least one direction, such as the axial direction (illustrated in Fig. 3) or the radial direction (illustrated in Fig. 4). As used herein, the term "proximate" means close enough that tissue thickness measurement element 62 can detect tissue interface(s) with an acceptable signal-to-noise ("S/N") ratio. The measured thickness may be associated with the position of distal end portion 60 (as measured by localization elements 52, 54, and/or 56) via a suitable controller, such as computer 20, operably coupled to tissue thickness measurement element 62. As used herein, the term "data point" refers to a set or matrix of values that associates a thickness measurement along a particular direction with the corresponding location of distal end portion 60 of catheter 13 (*e.g.*, (x, y, z) or (x, y, z $\Theta_1$, $\Theta_2$, $\Theta_3$)) for that measured thickness. The data point may also include other parameters, such as sample time or information about multiple layers or subthicknessess of a measured tissue.

Typically, electrodes 52, 54, and 56 are employed to measure (x, y, z) coordinates, and thereby to deduce or compute $\Theta_1$, $\Theta_2$, and $\Theta_3$ of distal end portion 60 of catheter 13. This is especially desirable for a directional thickness measurement element 62, which may be capable of measuring a thickness along an axial beamline that is outside or beyond the face of the tip of catheter 13. In these aspects of the invention (*e.g.*, where the distal end portion 60 of catheter 13 is stood off from the tissue surface), the true spatial location of the thickness measurement may differ from the directly-measured (x, y, z) coordinates of electrodes 52, 54, and 56. The measured position and computed orientation of catheter 13 advantageously allows for a computation of the true spatial location of the thickness measurement for purposes of associating a position and a thickness as a data point. Thus, one of ordinary skill in the art will appreciate that the present teachings may be applied whether distal end portion 60 of catheter 13 is touching the tissue to be measured or stood off therefrom within a blood pool.

According to the invention, tissue thickness measurement element 62 includes an acoustic transducer 64, typically operable in a pulse-echo mode to measure the thickness of nearby tissue. Suitable acoustic transducers include, without limitation, piezoelectric transducers, magnetostrictive transducers, electrostatic transducers, microelectromechanical (MEMS) transducers, capacitive MEMS ultrasonic transducers (CMUTs), piezoelectric MEMS ultrasonic transducers (PMUTs), or any other transducers capable of emitting and receiving acoustic energy in a pulse-echo mode. The transducers may also be arranged in an array, such as a linear phased array. One or more electrical leads 66 may connect to acoustic transducer 64 to carry power and/or data signals thereto and therefrom.

Many types and arrangements of transducers are suitable for use in connection with the present invention. For example, both directional disc-shaped transducers and omnidirectional tubular and/or cylindrical transducers may be employed. (As used herein, the term "directional" refers to a transducer that transmits and receives an acoustic beam substantially along only a single direction.) Likewise, transducers that articulate or rotate within distal end portion 60 of catheter 13, such that they can observe along multiple directions from within a stationary catheter, are contemplated. The present invention may also utilize transducers having differently-oriented portions that are electrically switchable. Also contemplated are transducers that slide along the catheter to provide a diversity of viewpoints. One of ordinary skill in the art will appreciate from this disclosure how to select and arrange one or more suitable transducers on or within catheter 13.

Typically, transducer 64 will perform pulse-echo pinging at a frequency between about 3 MHz and about 20 MHz, with higher frequencies being employed for thinner tissues requiring more accuracy and less penetration. For cardiac wall thickness, suitable transducers operate between about 4 MHz to about 12 MHz.

Typically, transducer 64 will include a piezocrystal. Acoustic transducer 64 may optionally be utilized in conjunction with one or more acoustic matching layers 68 on the crystal frontside. The ordinary artisan will also recognize the desirability of backing acoustic transducer 64 with an attenuative backing material 70 such that backwards-going acoustic energy is rapidly damped out to prevent acoustic ringing. Acoustic transducer 64 may also be coupled to an electrical matching circuit or electrical matching element (not shown), for example via hot and ground leads 66. The electrical matching circuit or element may conveniently be provided in the control handle or connector (neither shown) of catheter 13.

According to the invention, acoustic transducer 64 emits and receives acoustic energy through an acoustic standoff. For example, Fig. 3 depicts an acoustic standoff 72 positioned distally of acoustic transducer 64 that separates acoustic transducer 64 from the tip of catheter 13. Likewise, Fig. 4 depicts acoustic standoffs 72' that are positioned radially outward from acoustic transducers 64'. Acoustic standoffs desirably put a propagation time delay between the relatively larger driving pulse and the relatively smaller near field reception echoes such that the near field echoes are more easily discerned. Furthermore, the distal end portion 60 of catheter 13 is relatively rigid where acoustic transducer 64 is carried, as this permits the orientation of distal end portion 60 to be deduced from the measured locations of the electrodes (or other localization elements) thereon.

Fig. 3 depicts acoustic transducer 64 oriented to emit and receive acoustic energy in a direction substantially along the longitudinal axis 100 of catheter 13. It should be understood, however, that according to the invention, acoustic transducer 64 may be oriented to emit and receive acoustic energy at one or more angles to the longitudinal axis of catheter 13. For example, circular mapping catheters, such as the LIVEWIRE SPIRAL HP™ catheter of St. Jude Medical, Atrial Fibrillation Division, Inc., are often used in diagnostic and therapeutic procedures in the pulmonary veins. In such a circular mapping catheter, it may be desirable to orient one or more acoustic transducers to emit and receive acoustic energy along a radius of the curve. Such an arrangement is visible in the cutaway section of a distal end portion 60' of a catheter 13' illustrated in Fig. 4.

The use of catheter 13 will be described in connection with creation of a map of cardiac tissue thickness. It should be understood, however, that the principles disclosed herein may be applied to mapping the thickness of any tissue as well as the thickness of other structures, including the sizes or dimensions of fluid cavities.

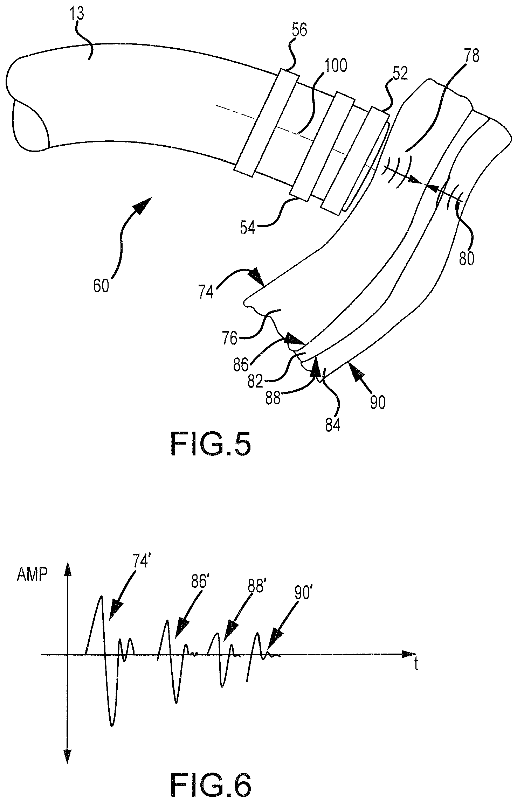

Distal end portion 60 of catheter 13 is introduced into a portion of a patient's heart, such as a heart chamber or pulmonary vein ostium, typically by navigating catheter 13 through the patient's vasculature. As shown in Fig. 5, distal end portion 60 may then be brought either in contact with or sufficiently close to endocardial surface 74 to enable measurement of the thickness of the myocardium 76. The term "sufficiently close" means close enough to receive a reasonable detectable echo from the tissue interface/surface to be detected. Depending on the pulsing voltage and beamshape, "sufficiently close" may be between a few millimeters to over a centimeter.

The position (*e.g.*, the (x, y, z) coordinates) of distal end portion 60 of catheter 13 may be measured using localization system 8 (*e.g.*, localization field generators 12/14, 18/19, and 16/22 in conjunction with localization elements 52, 54, and 56). As described above, the orientation of distal end portion 60 may be deduced from the measured locations of multiple electrodes (*e.g.*, electrodes 52, 54, and 56) that are closely spaced on a relatively rigid segment of catheter 13. Alternatively, certain localization systems 8 may directly measure both the position and the orientation of distal end portion 60.

Fig. 5 also illustrates outgoing or transmitted pulses 78 of acoustic energy that are emitted by acoustic transducer 64 towards myocardium 76; returning echoes 80 of acoustic energy from myocardium 76 are received by acoustic transducer 64. One of ordinary skill in the art will appreciate that transmitted outgoing acoustic pulses 78 will at least partially reflect backwards towards acoustic transducer 64 as echoes 80 upon encountering interfaces between different materials or tissue layers, such as the respective interfaces between the blood pool, myocardium 76, pericardial fluid 82, and pericardium 84 (*e.g.*, endocardial surface 74 and pericardial surface 90). As described below, these echoes 80 can be interpreted as information about the distance to the tissue interface/surface and therefore the thickness of myocardium 76, as well as about the thickness of the pericardial fluid 82 and the pericardium 84.

By measuring the delay time t of each interface's reflection and using the acoustic velocity $v_s$ of blood and tissue at 37 degrees C (*e.g.*, about 1520 m/sec), the distance *D* to each interface/tissue surface can be computed according to the equation $D = \frac{1}{2} v_s t$, wherein the factor of ½ accounts for the round-trip of acoustic waves propagating outwards and then returning inwards. The thickness of a given tissue can then be computed as the difference in distance between its near and far detected interfaces or surfaces.

Fig. 6 is a representative graph of pulse-echo strength versus time. Each peak 74', 86', 88', and 90' in Fig. 6 corresponds to an acoustic echo 80 reflecting from an interface between distinct materials (*e.g.*, myocardium 76 and pericardial fluid 82). By using the transmission velocity of the acoustic energy emitted by acoustic transducer 64 in the tissue being measured, the time at which each echo 80 is received can be converted into tissue thickness measurements as described above, such that the x-axis of Fig. 6 could also be representative of distance to the tissue interface/surface from the transducer. Thus, one of ordinary skill in the art will appreciate how to interpret a graph of echo amplitude versus time as information concerning the depth of the following interfaces, and therefore the thicknesses of the various layers: (1) the endocardial surface 74 (peak 74'); (2) the interface 86 between myocardium 76 and pericardial fluid 82 (peak 86'); (3) the interface 88 between pericardial fluid 82 and pericardium 84 (peak 88'); and (4) the pericardial surface 90 (peak 90'). Information about the thickness of the cardiac tissue may be associated with the measured location of distal end portion 60 of catheter 13 as a data point. The tissue thickness data thus paired with location data can, as described in further detail below, be displayed in a textual or graphical manner (*e.g.*, via presentation of a color-coded tissue thickness map), for example in conjunction with a chamber geometry generated using localization system 8.

One of ordinary skill in the art will recognize that the tissue thickness measured will be a function of the angle at which the acoustic energy is emitted and received relative to the tissue layer's orientation to the acoustic beam. Tissue thickness measurements as described herein, however, whether taken orthogonal to the tissue surface or not, detect the location of the tissue interfaces along that beamline or direction of energy delivery. Of course, even if the tip of catheter 13 is placed orthogonal to the tissue surface, deeper interfaces may not be orthogonally oriented.

Thus, at least two thickness measurements can be reported for a given measurement location. The first is referred to herein as "projected thickness" or simply "thickness," and refers to the thickness measured along a specific beamline (*e.g.*, the thickness along whatever angle the beamline penetrates the tissue). The second is referred to herein as the "minimum thickness," which is the thickness measured along the shortest path through the layer at that measurement location, and may be useful in certain surgical applications.

It should be understood that, by "sweeping" the beam angle with respect to the tissue (*e.g.*, by moving distal end portion 60 of catheter 13 within the blood pool or rotating or sliding it along the tissue surface), one can capture a number of projected thicknesses as well as the minimum thickness. If one forms a 3D layered or thickness-depicting spatial model of the heart using the inventive catheter, one can use that established model formed from a large set of projected thicknesses to post-compute or visually observe (*e.g.*, via colorization) any desired minimum thickness.

In another aspect distal end portion 60 of catheter 13 is placed into a blood pool (*e.g.*, within a heart chamber) and simply allowed to move with the motion of the blood pool. As distal end portion 60 of catheter 13 "floats" within the blood pool, its beam angle sweeps relative to the tissue, enabling it to detect cardiac surfaces and measure a plurality of thicknesses along several differently positioned and/or oriented beamlines. By associating these detected surfaces or interfaces and with the positions and orientations at which they were detected and measured, a tissue map or 3D model of a portion of the heart may be generated.

It is therefore desirable to measure both the position and orientation of catheter 13, typically by utilizing multiple localization elements at distal end portion 60. One suitable method for determining the position and orientation of a catheter, sheath, or other probe carrying multiple localization elements is disclosed in United States application no. 12/347,271.

By associating detected tissue interface distances relative to acoustic transducer 64 with the corresponding localization information (*e.g.*, position and orientation of distal end portion 60 of catheter 13), the maps and graphical representations of tissue thickness described herein may be spatially constructed.

In some aspects a plurality of spatially situated tissue interface data points (referred to herein as "geometry points") are generated by repeatedly measuring the location/orientation of distal end portion 60 of catheter 13 and the projected distances of proximate cardiac tissue as catheter 13 is moved through or relative to the portion of the heart (*e.g.*, as catheter 13 sweeps through the heart chamber). This plurality of geometry data points may be stored in a memory device and processed by a mapping processor, such as a software program running on computer 20 or a dedicated mapping processor, into a map of cardiac tissue thickness. A graphical representation of the map of cardiac tissue thickness may then be output on a display 23.

In other aspects a projected tissue thickness or interface location is measured repeatedly at a single location within the heart at several times during the patient's cardiac cycle so as to capture a plurality of cardiac cycle data points. For example, the measurement could be gated to an ECG such that the measurements are typically taken when the heart has the same shape and the catheter is pointed in the same direction (*e.g.*, at the same point in the cardiac cycle from cycle to cycle). Alternatively, the measurements could be taken at different times during the cardiac cycle. The plurality of cardiac cycle data points may be used to generate a map of how cardiac tissue thickness changes during the cardiac cycle. It is also contemplated that the series of projected tissue thicknesses may be used to derive an average (*e.g.*, mean), minimum, or maximum thickness of the cardiac tissue at a particular location or region on the heart (as measured by localization system 8). One or more of these thickness states, such as the average thickness, can then be associated with the measured spatial-model surface location as a geometry data point. By repeating the process at a plurality of locations within the heart, for example by sweeping catheter 13 around the heart chamber, a plurality of geometry data points can be generated. This plurality of geometry data points can be processed into a spatial tissue model associated with specific thickness states as described above. Graphical representations of any of the foregoing maps may, of course, be presented to a practitioner on display 23.

In some aspects, the graphical representation of the map of cardiac tissue thickness, average, maximum, or minimum cardiac tissue thickness vs. time or location, or changes in cardiac tissue thickness may be overlaid upon a three-dimensional (3D) model of the portion of the heart. The 3D model of the portion of the heart may be derived from any suitable imaging modality, including being generated from the measured locations included in the plurality of geometry data points collected during the thickness mapping operation described above. Alternatively, the 3D model may be a preexisting model based upon previously gathered data or imagery (*e.g.*, a CATSCAN or MRI image). It may also be desirable to overlay a graphical representation of electrophysiological data on the three-dimensional model of the portion of the heart and register them temporally and/or geometrically thereto.

Such maps and spatial graphical representations thereof may be employed for desirable purposes in connection with a variety of diagnostic and therapeutic procedures, and are particularly advantageous in connection with ablation procedures used to treat atrial and ventricular fibrillation and other rhythm disorders. For example, a practitioner may determine or modify an appropriate treatment protocol with reference to the tissue thickness information included in the map.

Alternatively, such a map may be used to execute an appropriate ablation treatment, either manually or automatically, by activating and deactivating ablation elements within the patient's heart. In this respect, it is contemplated that one or more of electrodes 17, 52, 54, and 56 may be used as ablation electrodes. Likewise, acoustic transducer 64 may be operated in an ablation mode to deliver ablating energy, such as high intensity focused ultrasound ("HIFU") energy, to tissue to be ablated. Alternatively, a separate ablation catheter, equipped with localization elements that permit its location with the patient's body to be measured using localization system 8, may be introduced into the patient's heart to carry out the ablation treatment. For example, it is contemplated that one or more devices according to the present invention may be used to detect and/or map the thinnest portion of a pulmonary vein ("PV") either epicardially or endocardially. Once the thinnest portion of the PV has been detected, a PV isolation lesion may be created either epicardially (*e.g.*, by lesioning about the PV trunk) or endocardially (*e.g.*, by lesioning about the PV ostia). Advantageously, as discussed above, for epicardial applications, acoustic transducer 64 may be operated in an ablation mode to deliver HIFU energy to ablate the pulmonary veins at their thinnest points. Included in the inventive scope is the operation of acoustic transducer 64 to both measure tissue thickness and ablate while situated at a particular tissue location. Such a dual-acting transducer may be abutted to the tissue surface directly during such thickness measurement and ablation.

Although several embodiments of this invention have been described above with a certain degree of particularity, those skilled in the art could make numerous alterations to the disclosed embodiments without departing from the scope of this invention. For example, to aid in navigating catheter 13 through the patient's vasculature to the target location, catheter 13 may be made steerable via the inclusion of one or more steering (or "pull") wires, such as disclosed in United States application no. 11/647,313.

Alternatively, catheter 13 may be navigated to its destination over a guidewire or through an introducer.

It is also contemplated that, as an alternative or an addition to the graphical representations of tissue thickness described above, the various data points may be displayed to a user of the inventive system in text form.

All directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the scope of the invention as defined in the appended claims.

## Claims

1. A device for mapping thickness of anatomical structures, comprising:

  an elongate catheter body having a distal end portion (60) including a rigid segment; a plurality of localization elements (52, 54, 56) carried by the rigid segment of the distal end portion (60) of the elongate catheter body to measure a location of the distal end portion (60) of the elongate catheter body via a localization system (8); at least one pulse-echo transducer (64) carried by the rigid segment of the distal end portion (60) of the elongate catheter body, wherein the at least one pulse-echo transducer (64) is adapted for emitting acoustic energy and receiving echo signals for measuring a thickness of a tissue proximate the distal end portion (60) of the elongate catheter body when the distal end portion (60) is stood off from the tissue within a blood pool or touching the tissue; and
  at least one acoustic standoff (72) positioned radially outward from the at least one pulse-echo transducer (64) with respect to a longitudinal axis of the elongate catheter body such that energy emitted and received by the at least one pulse-echo transducer (64) travels through the at least one acoustic standoff (72) thereby introducing a propagation time delay between driving pulses emitted by the at least one pulse-echo transducer and near field echos received by the at least one pulse-echo transducer such that the near field echos are more easily discerned; a controller configured to associate the measured location of the distal end portion (60) of the elongate catheter body with the measured thickness of the tissue proximate the distal end portion (60) of the elongate catheter body.

2. The device according to claim 1, wherein the localization elements (52, 54, 56) are configured to measure a location of the distal end portion (60) of the elongate catheter body within a non-ionizing localization field.

3. The device according to claim 1 or 2, wherein the localization elements (52, 54, 56) comprise at least one magnetic localization element configured to measure a location of the distal end portion (60) of the elongate catheter body within a magnetic localization field.

4. The device according to any one of claims 1 to 2, wherein localization elements (52, 54, 56) comprise at least one electrical localization element configured to measure a location of the distal end portion (60) of the elongate catheter body within an electric localization field.

5. The device according to any one of claims 1 to 4, wherein localization elements (52, 54, 56) comprise at least three localization elements.

6. The device according to any one of claims 1 to 5, wherein the at least one pulse-echo transducer (64) is oriented such that it emits and receives energy in a direction angled with respect to a longitudinal axis of the elongate catheter body.

7. The device according to any one of claims 1 to 6, wherein the transducer is operable to detect a distance to a tissue interface proximate the distal end portion of the catheter body and the controller is configured to determine a location of the tissue interface from the localization of the distal end portion (60) of the catheter body and the detected distance to the tissue interface.

8. A system for mapping cardiac tissue thickness, comprising:

a localization system (8);
an elongate catheter body having a distal end portion (60) including a rigid segment; a plurality of localization elements (52, 54, 56) carried by the rigid segment of the distal end portion (60) of the elongate catheter body to measure a location of the distal end portion (60) of the elongate catheter body within a portion of a heart using the localization system (8);
at least one ultrasonic transducer (64') carried by the rigid segment of the distal end portion (60) of the elongate catheter body, the at least one ultrasonic transducer (64') operable to emit acoustic energy and receive echo signals for measuring a thickness of a cardiac tissue proximate the distal end portion (60) of the elongate catheter body when the distal end portion (60) is stood off from the tissue within a blood pool or touching the tissue; and
at least one acoustic standoff (72') positioned radially outward from the at least one ultrasonic transducer (64') with respect to a longitudinal axis of the elongate catheter body such that energy emitted and received by the at least one ultrasonic transducer (64') travels through the at least one acoustic standoff (72) thereby introducing a propagation time delay between driving pulses emitted by the at least one pulse-echo transducer and near field echos received by the at least one pulse-echo transducer such that the near field echos are more easily discerned,

wherein the localization system (8) further comprises a controller configured to associate the measured location of the distal end portion (60) of the elongate catheter body with the measured thickness of the cardiac tissue proximate the distal end portion (60) of the elongate catheter body as a data point.

9. The system according to claim 8, wherein the localization system (8) is configured to generate an electric field and the localization elements (52, 54, 56) are configured to measure a characteristic of the electric field to determine the location of the distal end portion (60) of the elongate catheter body.

10. The system according to claim 8, wherein the localization system (8) is configured to generate a magnetic field and the localization elements (52, 54, 56) are configured to measure a characteristic of the magnetic field to determine the location of the distal end portion (60) of the elongate catheter body.

11. The system according to claim 8, wherein the localization system (8) comprises an acoustic localization system.

12. The system according to any one of claims 8 to 11, further comprising a display (23), wherein the localization system (8) is configured to output a three-dimensional image of the portion of the heart on the display (23) and the localization elements (52, 54, 56) make the distal end portion (60) of the elongate catheter body visible within the three-dimensional image.

13. The system according to any one of claims 8 to 12, further comprising:

a mapping processor that generates a map of cardiac tissue thickness from a plurality of data points; and
a display (23) configured to output a graphical representation of the map of cardiac tissue thickness overlaid upon a three-dimensional model of the portion of the heart,

14. The system according to claim 13, wherein the three-dimensional model of the portion of the heart is generated from the plurality of data points.

**Patentansprüche**

1. Vorrichtung zur Abbildung der Dicke von anatomischen Strukturen, mit:

einem länglichen Katheterkörper, der einen distalen Endbereich (60) mit einem starren Segment aufweist;
einer Mehrzahl von Ortungselementen (52, 54, 56), die von dem starren Segment des distalen Endbereichs (60) des länglichen Katheterkörpers getragen werden, um eine Position des distalen Endbereichs (60) des länglichen Katheterkörpers über ein Ortungssystem (8) zu messen;
mindestens einem Impulsechowandler (64), der von dem starren Segment des distalen Endbereichs (60) des länglichen Katheterkörpers getragen wird, wobei der mindestens eine Pulsechowandler (64) angepasst ist, akustische Energie zu emittieren und Echosignale zu empfangen, um eine Dicke eines Gewebes nahe dem distalen Endbereich (60) des länglichen Katheterkörpers zu messen, wenn der distale Endbereich (60) von dem Gewebe innerhalb eines Blutpools getrennt ist oder das Gewebe berührt; und
mindestens einem akustischen Abstandselement (72), das radial nach außen von dem mindestens einen Pulsechowandler (64) in Bezug auf eine Längsachse des länglichen Katheterkörpers derart angeordnet ist, dass die von dem mindestens einen Pulsechowandler (64) emittierte und empfangene Energie durch den mindestens einen Pulsechowandler (64) verläuft, wodurch eine Ausbreitungszeitverzögerung zwischen Anregungsimpulsen, die von dem mindestens einen Impulsechowandler ausgesendet werden, und Nahfeldechos, die von dem mindestens einen Impulsechowandler empfangen werden, eingeführt wird, so dass Nahfeldechos leichter zu erkennen sind; einer Steuerung, die konfiguriert ist, um die gemessene Position des distalen Endbereichs (60) des länglichen Katheterkörpers der gemessenen Dicke des Gewebes nahe dem distalen Endbereich (60) des länglichen Katheterkörpers zuzuordnen.

2. Vorrichtung nach Anspruch 1, bei der die Ortungselemente (52, 54, 56) konfiguriert sind zum Messen einer Position des distalen Endbereichs (60) des länglichen Katheterkörpers in einem nicht-ionisierenden Ortungsfeld.

3. Vorrichtung nach Anspruch 1 oder 2, bei der die Ortungselemente (52, 54, 56) mindestens ein magnetisches Ortungselement aufweisen, das konfiguriert ist zum Messen einer Position des distalen Endbereichs (60) des länglichen Katheterkörpers in einem magnetischen Ortungsfeld.

4. Vorrichtung nach einem der Ansprüche 1 und 2, bei der die Ortungselemente (52, 54, 56) mindestens einen elektrischen Ortungsgerät aufweisen, das konfiguriert ist zum Messen einer Position des distalen Endbereichs (60) des länglichen Katheterkörpers in einem elektrischen Ortungsfeld.

5. Vorrichtung nach einem der Ansprüche 1 bis 4, bei der die Ortungselemente (52, 54, 56) mindestens drei Ortungselemente aufweisen.

6. Vorrichtung nach einem der Ansprüche 1 bis 5, bei der der mindestens eine Pulsechowandler (64) derart ausgerichtet ist, dass er Energie in einer Richtung abgibt und empfängt, die bezüglich einer Längsachse des länglichen Katheterkörpers geneigt ist.

7. Vorrichtung nach einem der Ansprüche 1 bis 6, bei der der Wandler betreibbar ist, zum Detektieren eines Abstands zu einer Gewebeschnittstelle nahe dem distalen Endbereich des Katheterkörpers, und die Steuerung konfiguriert ist zum Bestimmen einer Position der Gewebeschnittstelle von der Position des distalen Endbereichs (60) des Katheterkörpers und dem erfassten Abstand zu der Gewebeschnittstelle.

8. System zur Abbildung der Dicke von Herzgewebe, mit:

einem Ortungssystem (8); einem länglichen Katheterkörper, der einen distalen Endbereich (60) mit einem starren Segment aufweist; einer Mehrzahl von Ortungselementen (52, 54, 56), die von dem starren Segment des distalen Endbereichs (60) des länglichen Katheterkörpers getragen werden, um eine Position des distalen Endbereichs (60) des länglichen Katheterkörpers in eines Bereichs eines Herzens mittels des Ortungssystems (8) zu messen; mindestens einem Ultraschallwandler (64'), der von dem starren Segment des distalen Endbereichs (60) des länglichen Katheterkörpers getragen wird, wobei der mindestens eine Ultraschallwandler (64') betreibbar ist, um akustische Energie abzugeben und Echosignale zu empfangen, um eine Dicke von Herzgewebe in der Nähe des distalen Endbereichs (60) des länglichen Katheterkörpers zu messen, wenn der distale Endbereich (60) von dem Gewebe innerhalb eines Blutpools getrennt ist oder das Gewebe berührt; und mindestens einem akustischen Abstandselement (72'), das von dem mindestens einen Ultraschallwandler (64') in Bezug auf eine Längsachse des Katheterkörpers radial nach außen derart angeordnet ist, dass die von dem mindestens einen Ultraschallwandler (64') abgegebene und empfangene Energie durch das mindestens eine Abstandselement (72') verläuft, wodurch eine Ausbreitungszeitverzögerung zwischen Anregungsimpulsen, die von dem mindestens einen Impulsechowandler ausgesendet werden, und Nahfeldechos, die von dem mindestens einen Impulsechowandler empfangen werden, eingeführt wird, so dass Nahfeldechos leichter zu erkennen sind, wobei das Ortungssystem (8) ferner eine Steuerung aufweist, die konfiguriert ist zum Zuordnen der gemessene Position des distalen Endabschnitts (60) des länglichen Katheterkörpers zu der gemessenen Dicke von Herzgewebe in der Nähe des distalen Endabschnitts (60) des länglichen Katheterkörpers als ein Datenpunkt.

9. System nach Anspruch 8, bei dem das Ortungssystem (8) konfiguriert ist zum Erzeugen eines elektrisches Feld, und die Ortungselemente (52, 54, 56) konfiguriert sind zum Messen einer Eigenschaft des elektrischen Feldes, um die Position des distalen Endabschnitts (60) des länglichen Katheterkörpers zu bestimmen.

10. System nach Anspruch 8, bei dem das Ortungssystem (8) konfiguriert ist zum Erzeugen eines Magnetfeld, und die Ortungselemente (52, 54, 56) konfiguriert sind zum Messen einer Eigenschaft des Magnetfelds, um die Position des distalen Endabschnitts (60) des länglichen Katheterkörpers zu bestimmen.

**11.** System nach Anspruch 8, bei dem das Ortungssystem (8) ein akustisches Ortungssystem aufweist.

**12.** System nach einem der Ansprüche 8 bis 11, ferner mit einer Anzeige (23), wobei das Ortungssystem (8) konfiguriert ist zum Erzeugen eines dreidimensionale Bilds des Bereichs des Herzens auf der Anzeige (23), und die Ortungselemente (52, 54, 56) den distalen Endbereich (60) des länglichen Katheterkörpers in dem dreidimensionalen Bild sichtbar machen.

**13.** System nach einem der Ansprüche 8 bis 12, ferner mit:

einen Abbildungsprozessor, der aus einer Mehrzahl von Datenpunkten eine Karte der Herzgewebedicke erzeugt; und
eine Anzeige (23), die konfiguriert ist zum Erzeugen einer grafischen Darstellung der Herzgewebedickenkarte, die einem dreidimensionalen Modell des Bereichs des Herzens überlagert ist.

**14.** System nach Anspruch 13, bei dem das dreidimensionale Modell des Bereichs des Herzen aus der Mehrzahl von Datenpunkten erzeugt wird.

**Revendications**

**1.** Dispositif de cartographie de l'épaisseur de structures anatomiques, comprenant :

un corps de cathéter allongé possédant une partie d'extrémité distale (60) comprenant un segment rigide ;
une pluralité d'éléments de localisation (52, 54, 56) supportés par le segment rigide de la partie d'extrémité distale (60) du corps de cathéter allongé pour mesurer un emplacement de la partie d'extrémité distale (60) du corps de cathéter allongé par l'intermédiaire d'un système de localisation (8) ;
au moins un transducteur d'écho par impulsions (64) supporté par le segment rigide de la partie d'extrémité distale (60) du corps de cathéter allongé, dans lequel l'au moins un transducteur d'écho par impulsions (64) est adapté pour émettre de l'énergie acoustique et recevoir des signaux d'écho afin de mesurer une épaisseur d'un tissu proche de la partie d'extrémité distale (60) du corps de cathéter allongé lorsque la partie d'extrémité distale (60) est séparée du tissu dans un pool sanguin intracardiaque ou touche le tissu ; et
au moins un élément d'interposition acoustique (72) positionné radialement vers l'extérieur à partir de l'au moins un transducteur d'écho par impulsions (64) par rapport à un axe longitudinal du corps de cathéter allongé de sorte que l'énergie émise et reçue par l'au moins un transducteur d'écho par impulsions (64) se déplace à travers l'au moins un élément d'interposition acoustique (72) introduisant ainsi un retard de temps de propagation entre des impulsions d'excitation émises par l'au moins un transducteur d'écho par impulsions et des échos en champ proche reçus par l'au moins un transducteur d'écho par impulsions de sorte que les échos en champ proche sont discernés plus facilement ;
un contrôleur configuré pour associer l'emplacement mesuré de la partie d'extrémité distale (60) du corps de cathéter allongé à l'épaisseur mesurée du tissu proche de la partie d'extrémité distale (60) du corps de cathéter allongé.

**2.** Dispositif selon la revendication 1, dans lequel les éléments de localisation (52, 54, 56) sont configurés pour mesurer un emplacement de la partie d'extrémité distale (60) du corps de cathéter allongé dans un champ de localisation non ionisant.

**3.** Dispositif selon la revendication 1 ou 2, dans lequel les éléments de localisation (52, 54, 56) comprennent au moins un élément de localisation magnétique configuré pour mesurer un emplacement de la partie d'extrémité distale (60) du corps de cathéter allongé dans un champ de localisation magnétique.

**4.** Dispositif selon l'une quelconque des revendications 1 et 2, dans lequel les éléments de localisation (52, 54, 56) comprennent au moins un élément de localisation électrique configuré pour mesurer un emplacement de la partie d'extrémité distale (60) du corps de cathéter allongé dans un champ de localisation électrique.

**5.** Dispositif selon l'une quelconque des revendications 1 à 4, dans lequel les éléments de localisation (52, 54, 56) comprennent au moins trois éléments de localisation.

**6.** Dispositif selon l'une quelconque des revendications 1 à 5, dans lequel l'au moins un transducteur d'écho par impulsions (64) est orienté de telle sorte qu'il émet et reçoit de l'énergie dans une direction inclinée par rapport à un axe longitudinal du corps de cathéter allongé.

**7.** Dispositif selon l'une quelconque des revendications 1 à 6, dans lequel le transducteur est utilisable pour détecter une distance jusqu'à une interface de tissu proche de la partie d'extrémité distale du corps de cathéter et le contrôleur est configuré pour déterminer un emplacement de l'interface de tissu à partir de la localisation de la partie d'extrémité distale (60) du corps de cathéter et de la distance détectée jusqu'à l'interface de tissu.

**8.** Système de cartographie de l'épaisseur d'un tissu cardiaque, comprenant :

un système de localisation (8) ;
un corps de cathéter allongé possédant une partie d'extrémité distale (60) comprenant un segment rigide ;
une pluralité d'éléments de localisation (52, 54, 56) supportés par le segment rigide de la partie d'extrémité distale (60) du corps de cathéter allongé pour mesurer un emplacement de la partie d'extrémité distale (60) du corps de cathéter allongé dans une partie d'un coeur au moyen du système de localisation (8) ;
au moins un transducteur à ultrasons (64') supporté par le segment rigide de la partie d'extrémité distale (60) du corps de cathéter allongé, l'au moins un transducteur à ultrasons (64') étant utilisable pour émettre de l'énergie acoustique et recevoir des signaux d'écho afin de mesurer une épaisseur d'un tissu cardiaque proche de la partie d'extrémité distale (60) du corps de cathéter allongé lorsque la partie d'extrémité distale (60) est séparée du tissu dans un pool sanguin intracardiaque ou touche le tissu ; et
au moins un élément d'interposition acoustique (72') positionné radialement vers l'extérieur à partir de l'au moins un transducteur à ultrasons (64') par rapport à un axe longitudinal du corps de cathéter allongé de sorte que l'énergie émise et reçue par l'au moins un transducteur à ultrasons (64') se déplace à travers l'au moins un élément d'interposition acoustique (72) introduisant ainsi un retard de temps de propagation entre des impulsions d'excitation émises par l'au moins un transducteur d'écho par impulsions et des échos en champ proche reçus par l'au moins un transducteur d'écho par impulsions de sorte que les échos en champ proche sont discernés plus facilement,
dans lequel le système de localisation (8) comprend en outre un contrôleur configuré pour associer l'emplacement mesuré de la partie d'extrémité distale (60) du corps de cathéter allongé à l'épaisseur mesurée du tissu cardiaque proche de la partie d'extrémité distale (60) du corps de cathéter allongé comme un point de données.

**9.** Système selon la revendication 8, dans lequel le système de localisation (8) est configuré pour générer un champ électrique et les éléments de localisation (52, 54, 56) sont configurés pour mesurer une caractéristique du champ électrique afin de déterminer l'emplacement de la partie d'extrémité distale (60) du corps de cathéter allongé.

**10.** Système selon la revendication 8, dans lequel le système de localisation (8) est configuré pour générer un champ magnétique et les éléments de localisation (52, 54, 56) sont configurés pour mesurer une caractéristique du champ magnétique afin de déterminer l'emplacement de la partie d'extrémité distale (60) du corps de cathéter allongé.

**11.** Système selon la revendication 8, dans lequel le système de localisation (8) comprend un système de localisation acoustique.

**12.** Système selon l'une quelconque des revendications 8 à 11, comprenant en outre un dispositif d'affichage (23), dans lequel le système de localisation (8) est configuré pour produire une image tridimensionnelle de la partie du coeur sur le dispositif d'affichage (23) et les éléments de localisation (52, 54, 56) rendent la partie d'extrémité distale (60) du corps de cathéter allongé visible dans l'image tridimensionnelle.

**13.** Système selon l'une quelconque des revendications 8 à 12, comprenant en outre :

un processeur de cartographie qui génère une carte de l'épaisseur de tissu cardiaque à partir d'une pluralité de points de données ; et
un dispositif d'affichage (23) configuré pour produire une représentation graphique de la carte de l'épaisseur du tissu cardiaque superposée sur un modèle tridimensionnel de la partie du coeur.

**14.** Système selon la revendication 13, dans lequel le modèle tridimensionnel de la partie du coeur est généré à partir de la pluralité de points de données.

FIG.1

FIG.2

FIG.3

FIG.4

FIG.5

FIG.6

## REFERENCES CITED IN THE DESCRIPTION

**Patent documents cited in the description**

- US 6285898 B1
- US 2003013958 A1
- EP 1820464 A1
- US 5373849 A
- WO 9507657 A1
- US 2004073110 A1
- US 20040254437 A1
- US 11227580 B
- US 6990370 B
- US 6978168 B
- US 6947785 B
- US 6939309 B
- US 6728562 B
- US 6640119 B
- US 5983126 A
- US 5697377 A
- US 11967214 B
- US 12347271 B
- US 11647313 B